# EUROPÄISCHE PATENTSCHRIFT

(11) **EP 0 359 158 B1**
(45) Veröffentlichungstag und Bekanntmachung des Hinweises auf die Patenterteilung: **22.12.1993**
(21) Anmeldenummer: 89116722.3
(22) Anmeldetag: 09.09.1989
(51) Int. Cl.: G01N 33/18

(54) **Gerät und Verfahren zur Bestimmung von in Wasser enthaltenden Gasen und/oder in Gase überführbaren Substanzen**
Apparatus and method for determining the presence of gases or gasifiable substances in water
Appareil et procédé pour la détermination dans l'eau de gaz ou de substances gazéifiables

(30) Priorität: 16.09.1988 DE 3831476; 11.08.1989 DE 3926566
(43) Veröffentlichungstag der Anmeldung: 21.03.1990
(73) Patentinhaber: Solvay Umweltchemie GmbH, D-30173 Hannover (DE)
(72) Erfinder: Vorlop, Klaus Dieter, D-3300 Braunschweig (DE); Sell, Michael, D-3150 Peine (DE); Mehlitz, Dieter, D-3302 Cremlingen 5(Hemkenrode) (DE); Bennöhr, Thomas, D-3300 Braunschweig (DE)
(74) Vertreter: Lauer, Dieter, Dr.

(56) Entgegenhaltungen:
- FR-A- 2 415 299
- GB-A- 2 080 538
- ANAL. CHEMISTRY, Band 47, Nr. 3, März 1975; J. MERTENS et al., Seiten 522-526#

## Beschreibung

Die vorliegende Erfindung betrifft ein Gerät zur Bestimmung von in Wasser enthaltenen Gasen oder in Gase überführbaren Substanzen. Insbesondere betrifft die vorliegende Erfindung ein Gerät und ein Verfahren zur Bestimmung des Ammoniak/Ammoniumgehaltes in wäßrigen Systemen.

In anal. Chemistry, 47 (1975), Seiten 522 - 526, wird ein Analyseverfahren zur Nitratbestimmung in ammoniumhaltigem Wasser beschrieben, bei welchem Nitrat mit Devarda-Legierung reduziert und anschließend der Ammoniakgehalt der Reaktionslösung mit Hilfe von Meßelektroden bestimmt wird.

Die Bestimmung von Ammoniak und Ammonium nimmt in der Wasseranalyse, z.B. bei der Überwachung der Reinheit von Grund- und Oberflächenwasser sowie auch in der Analyse von Industrieabwässern einen wichtigen Platz ein. Ammonium-Stickstoff ist in fast allen Abwässern enthalten und liegt entsprechend dem pH-Wert des Wassers als NH₄⁺-Ion oder als gelöstes Ammoniak vor. In Grund- oder Oberflächenwasser vorliegendes Ammonium ist oft ein Abbauprodukt organischer Amine und ist somit ein Indiz für die Verunreinigung des Wassers mit organischen, möglicherweise hygienisch und gesundheitlich bedenklichen Stoffen.

Aufgabe der vorliegenden Erfindung ist es, ein Gerät zu entwickeln, mit welchem im Wasser enthaltene Gase und/oder leicht in Gase überführbare Substanzen in einfacher Weise und mit nur geringem Zeitaufwand nachgewiesen und quantitativ bestimmt werden können. Insbesondere ist es Aufgabe der vorliegenden Erfindung, ein Gerät und ein Verfahren zu entwickeln, welches eine weitgehend automatisierte Durchführung der Ammoniak/Ammonium-Bestimmung in Wasser ermöglichen.

Es wurde nun ein Gerät zur Bestimmung von Gasen und/oder in Gase überführbaren Substanzen in Wasser, und insbesondere ein Gerät und ein Verfahren zur Bestimmung von Ammoniak/Ammonium in Wasser gefunden, welche halbkontinuierlich und weitgehend automatisch arbeiten können, und welche insbesondere zur Analyse von Grundwässern, Flußwässern, Quellwässern oder Abwässern, beispielsweise in kommunalen oder industriellen Kläranlagen oder im Laborbetrieb oder zur Überwachung der Wasserqualität im Rahmen der Herstellung von Brauchwässern für die Lebensmittel- oder Getränkeindustrie oder von Trinkwasser verwendbar sind.

Insbesondere betrifft die Erfindung ein Verfahren zur halbkontinuierlich und automatisch durchführbaren Bestimmung des Ammoniak- und/oder Ammoniumgehaltes in Wasser, dadurch gekennzeichnet, daß man
a') das zu untersuchende Wasser über eine mit einem Ventil versehene Zuleitung in einen verschließbaren mit Flüssigkeits- und Gasein und -auslässen versehenen Dosierbehälter bis zu einem einer gewünschten Probenmenge entsprechenden Füllstand leitet,
b') in den das Wasser enthaltenden Dosierbehälter über eine weitere mit einem Ventil versehene Zuleitung eine verdünnte wäßrige Alkalimetallhydroxidlösung einleitet bis zur Erreichung eines Gesamtfüllstandes, welcher einer zur Einstellung eines pH-Wertes von mindestens 10,5, vorzugsweise mindestens 12, ausreichenden Zugabe an Alkalimetallhydroxid entspricht,
c') die alkalisch gestellte Wasserprobe aus dem Dosierbehälter über eine mit einem Ventil versehene Verbindungsleitung in eine mit Flüssigkeits- und Gasein- und -auslässen versehene Meßzelle leitet, in welcher eine NH₃-sensitiver Sensor, vorzugsweise eine NH₃-sensitive Meßelektrode angebracht ist,
d') in der Meßzelle eine konstante Temperatur im Bereich von 10 bis 50 °C aufrechterhält und in dem Meßbereich der Meßzelle eine konstante NH₃-Konzentration sich einstellen läßt,
e') die konstante NH₃-Konzentration in dem Meßbereich mißt und anschließend die alkalisierte Wasserprobe aus der Meßzelle ableitet und
f') aus dem gemessenen Wert durch Vergleich mit unter gleichen Bedingungen erhaltenen Eichwerten den Ammoniak und/oder Ammoniumgehalt des Wassers bestimmt.

Diese halbkontinuierlich, automatisch durchführbare Bestimmungsmethode kann unter Verwendung einer Meßzelle, in welcher ein NH₃-sensitiver Sensor in die zu messende Flüssigkeit hineinragt, durchgeführt werden. Es kann jedoch auch so gearbeitet werden, daß man die alkalisch gestellte Wasserprobe aus dem Dosierbehälter über eine mit einem Ventil versehene Verbindungsleitung in den unteren Bereich einer mit Flüssigkeits- und Gasein- und -auslässen versehenen Meßzelle überführt, in deren oberem Bereich ein NH₃-sensitiver Sensor, vorzugsweise eine NH₃-sensitive Meßelektrode, so angebracht ist, daß sie nicht mit der Flüssigkeit in Berührung kommt, in der Meßzelle eine konstante Temperatur im Bereich von 10 bis 50 °C aufrechterhält und in dem Gasraum der Meßzelle eine konstante NH₃-Konzentration sich einstellen läßt, und die konstante NH₃-Konzentration in dem Gasraum der Meßzelle mißt und anschließend die alkalisierte Wasserprobe aus der Meßzelle abführt.

Ferner ist Gegenstand der Erfindung ein Meßgerät zur Bestimmung von in Wasser gelösten GAsen und/oder in Gase überführbaren Substanzen, gekennzeichnet durch
a) einen verschließbaren Dosierbehälter D zur Aufnahme einer Probe des zu untersuchenden Wassers und allfälliger Reagenzlösungen, welcher mit Füllstandsanzeigern oder Füllstandssensoren versehen ist, in seinem unteren Bereich einen Flüssigkeitseinlaß für die zu untersuchende Wasserprobe, allfällige Reagenzlösungen und Eichlösungen und einen Flüssigkeitsauslaß besitzt und in seinem oberen Bereich einen Einlaß und einen Auslaß für Gas, insbesondere Luft oder ein Inertgas, besitzt.
b) mehrere, beispielsweise 2 bis 5, mit dem Flüssigkeitseinlaß des Dosierbehälters D verbundene mit Ventilen, z.B. V1, V2, V3, versehene Flüssigkeitszuleitungen, z.B. L1, L2, L3, für die zu untersuchende Wasserprobe, allfällige Reagenzlösungen und Eichlösungen.
c) eine verschließbare, mit einer Temperaturmeß- und -Regeleinrichtung T und einen Sensor S für das zu bestimmende Gas versehene Meßzelle M, welche einen zur Aufnahme der Flüssigkeitsmenge aus dem Dosierbehälter eingerichteten Bereich mit einem Flüssigkeitseinlaß und einem Flüssigkeitsauslaß besitzt und mit einem Einlaß und einem Auslaß für Gas, insbesondere Luft oder ein Inertgas versehen ist,
d) eine mit einem Ventil V4 versehene Flüssigkeitsleitung L4, welche den Flüssigkeitsauslaß des Dosierbehälters D mit dem Flüssigkeitseinlaß der Meßzelle M verbindet,
e) eine Saugdruckpumpe SP für Luft oder ein Inertgas, welche über jeweils mit einem Ventil V5, V6 versehene Gaszu- und -ableitungen L5, L6 be- bzw. entlüftbar ist, und welche auf ihrer Saugseite über eine von der Gaszuleitung L5 zwischen deren Ventil V5 und der Saug/Druckpumpe SP abzweigende mit einem Ventil V7 versehene Gasleitung L7 mit dem Gasauslaß des Dosierbehälters D und auf ihrer Druckseite über von der Gasableitung L6 zwischen deren Ventil V6 und der Saugdruckpumpe SP abzweigende jeweils mit Ventilen V8, V9 versehenen Gasleitungen L8, L9 mit dem Gaseinlaß des Dosierbehälters D und dem Gaseinlaß der Meßzelle M verbunden ist,
f) eine mit dem Gasauslaß der Meßzelle M verbundene mit einem Ventil V10 versehene Gasableitung L10
g) eine mit dem Flüssigkeitsauslaß der Meßzelle M verbundene mit einem Ventil V11 versehene Flüssigkeitsableitung L11

Die Gaszuleitung L5 zur Saug/Druckpumpe SP kann mit der Außenluft und/oder mit einem Inertgasbehälter verbunden sein. Die Gasableitung L 10 oder eine mit einem Ventil versehene Abzweigung davon können auch über eine zwischen dem Ventil V5 und der Saug/Druckpumpe SP liegende Abzweigung der Gasleitung L5 mit der Saugseite der Saug/Druckpumpe SP verbunden sein. Von der vom Gasauslaß des Dosierbehälters D wegführenden Gasleitung L7 kann eine weitere mit einem Ventil versehene Gasableitung abzweigen.

Flüssigkeitseinlaß und Flüssigkeitsauslaß können im Dosierbehälter D und in der Meßzelle M jeweils zu einer Öffnung zusammengefaßt sein. Das gleiche trifft auch für die jeweiligen Gasein- und -auslässe zu.

Der Gassensor des Gerätes kann mit einem Aufzeichnungs- und/oder Umrechnungsgerät verbunden sein, welches die vom Gassensor S gemessene Konzentration des bestimmten Gases aufzeichnen und/oder daraus im Vergleich mit Eichwerten die Konzentration des betreffenden Gases oder der dieses Gases generierenden Substanz in der untersuchten Wasserprobe berechnen kann. Die Gas- und Flüssigkeitsventile des Gerätes können elektronisch steuerbar sein. Die Meßzelle ist zweckmäßig mit einem Rührer versehen und kann gewünschtenfalls eine Gaseinleitung in den Flüssigkeitsbereich besitzen. Um einen automatischen Ablauf des Analysenvorgangs zu ermöglichen, können die einzelnen Aggregate (Ventile, Pumpe, Gassensor und Rührer der Meßzelle, Füllstandssensoren des Dosierbehälters etc.) mit einem Mikroprozessor verbunden und durch diesen über ein fest installiertes Programm gesteuert werden.

Beispielsweise kann das Gerät zur automatischen halbkontinuierlichen Durchführung der Bestimmung über einen Mikroprozessor mit fest installiertem Programm so gesteuert werden, daß mit Hilfe der Saug/Druckpumpe in sich wiederholenden Zyklen jeweils zunächst bei geöffneten Ventilen der jeweiligen Flüssigkeitszuleitungen zum Dosierbehälter und geschlossenem Ventil der Verbindungsleitung zur Meßzelle das zu untersuchende Wasser und eine allfällige Reagenzlösung bis zu im Programm vorgesehenen Füllständen in den Dosierbehälter eingesaugt werden, sodann bei geschlossenen Ventilen der Flüssigkeitszuleitungen und geöffnetem Ventil in der Verbindungsleitung die Flüssigkeit aus dem Dosierbehälter in die Meßzelle gedrückt wird, darin verbleibt, bis sich in dem Meßbereich der Meßzelle eine konstante Konzentration des zu messenden Gases eingestellt hat, und sodann die Flüssigkeit bei geschlossenem Verbindungsventil und geöffnetem Flüssigkeitsauslaß aus der Meßzelle gedrückt und diese mit Luft oder dem Inertgas gespült wird.

Gewünschtenfalls kann das Gerät zur automatischen halbkontinuierlichen Durchführung einer Qualitätskontrolle in fließendem Wasser zusätzlich eine mit einem Wassereinlaß, einem Wasserauslaß und einer Förderpumpe versehene Rohrleitung zur Durchleitung eines Teiles des fließenden Wassers besitzen, von welcher vor der Förderpumpe die Zuleitung für das Wasser zum Dosierbehälter abzweigt und in welche hinter der Förderpumpe eine mit dem Flüssigkeitsauslaß der Meßzelle verbundene Flüssigkeitsableitung einmündet.

Vorzugsweise ist das erfindungsgemäße Gerät mit einem NH₃-sensitiven Sensor, insbesondere eine NH₃-sensitiven Meßsonde ausgerüstet. Diese Ausführungsform ist zur halbkontinuierlichen, automatischen Bestimmung von Ammoniak/Ammonium in Wasser geeignet.

Bei Verwendung entsprechender anderer Gas-Sensoren kann das Gerät in gleicher Weise zur Bestimmung anderer in Wasser enthaltener Gase, z.B. Kohlendioxid, Kohlenmonoxid, Halogenen, flüchtigen Aminen oder Schwefelwasserstoff, oder von Substanzen welche leicht in eines der vorgenannten Gase überführbar sind, verwendet werden.

In einer Ausführungsorm des Gerätes kann der Gassensor S so angebracht sein, daß er in den zur Aufnahme der Flüssigkeit eingerichteten Bereich der Meßzelle hineinragt. Nach einer anderen Ausführungsform besitzt die Meßzelle einen zur Aufnahme der Flüssigkeitsmenge aus dem Dosierbehälter eingerichteten unteren Bereich mit einem Flüssigkeitseinlaß und einem Flüssigkeitsauslaß und einen zur Aufnahme von Gas, insbesondere Luft oder einem Inertgas, bestimmten oberen Bereich mit einem Gaseinlaß und einem Gasauslaß, in welchem ein Sensor S für das zu bestimmende Gas so angebracht ist, daß er nicht mit der zu untersuchenden Flüssigkeit in Berührung kommt.

Gewünschtenfalls kann die Meßzelle des Gerätes auch mit weiteren in den unteren das zu untersuchende Wasser aufnehmenden Bereich der Meßzelle eintauchenden Sensoren versehen sein, welche auf andere in dem Wasser enthaltene nicht flüchtige Stoffe, z.B. Nitrit oder Nitrat oder Fluorid, ansprechen. Mit einem solchen Gerät können gleichzeitig zwei Bestimmungen durchgeführt werden.

Nachfolgend wird die Verwendung des erfindungsgemäßen Gerätes am Beispiel des erfindungsgemäßen Verfahrens der Bestimmung von Ammonium/Ammoniak in Wasser dargestellt.

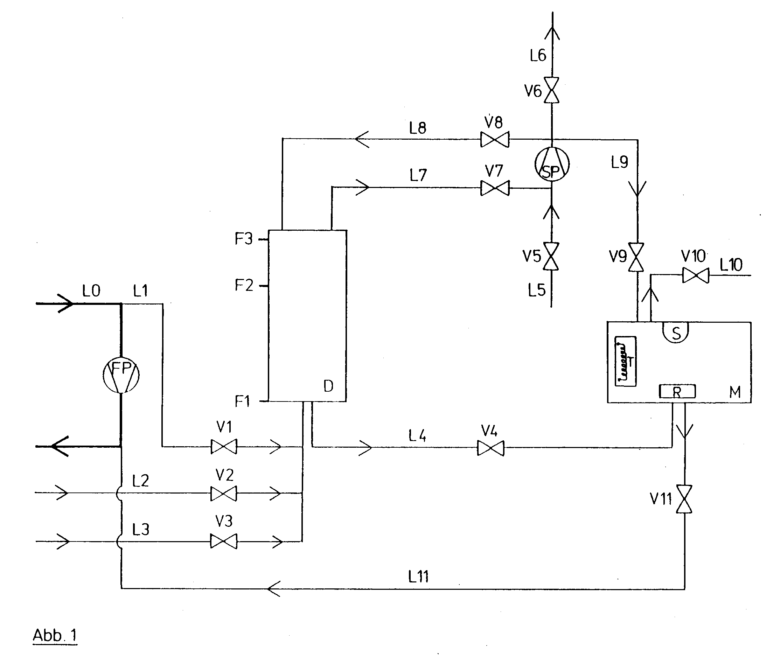

In Abbildung 1 ist ein zur halbkontinuierlichen automatischen Durchführung des Verfahrens geeignetes Gerät dargestellt.

Ein Teil des zu untersuchenden Wassers wird mittels einer Förderpumpe FP durch eine Rohrleitung L0 geleitet. Von dieser Rohrleitung führt eine mit einem Ventil V1 versehene Wasserzuleitung L1 zu dem Dosierbehälter D. Der Dosierbehälter ist mit mehreren unterschiedlich plazierten Füllstandssensoren F1, F2, F3 ausgerüstet. Ferner führt zu dem Dosierbehälter eine mit einem Ventil V2 versehene Zuleitung L2 für eine wäßrige Alkalimetallhydroxydlösung, insbesondere verdünnte Natronlauge. Ferner führt in den Dosierbehälter auch eine mit einem Ventil V3 versehene Zuleitung L3 zur Einleitung von einen vorbestimmten Ammoniumgehalt besitzenden Vergleichswässern. Der Transport der Flüssigkeit durch das Gerät erfolgt mit Hilfe einer Saug/Druckpumpe SP für Luft, welche über jeweils mit einem Ventil V5, V6 versehene Gaszu- und -ableitungen L5, L6 mit der Außenluft verbunden ist. Der geschlossene Dosierbehälter D ist über eine mit einem Ventil V8 versehene Gaseinleitung L8 und eine mit einem Ventil V7 versehene Gasableitung L7 mit der Saug/Druckpumpe SP für Luft verbunden. Das zu untersuchende Wasser bzw. ein Eichwasser und die verdünnte Natronlauge werden nach Öffnung der jeweiligen Ventile V1, V2, V3 bis zu dem gewünschten Füllstand in den Dosierbehälter D angesaugt. Hierbei sind die Ventile V7 und V6 der Gasableitungen L7 und L6 geöffnet und die Ventile V8 und V5 der Gaszuleitungen L8 und L5 und die Verbindung zur Meßzelle M geschlossen. Der Dosierbehälter D kann so ausgestaltet sein, daß er zwischen ca. 15 und 30 ml, vorzugsweise 17 bis 22 ml Flüssigkeit aufnehmen kann. Zweckmäßig übertrifft das Fassungsvermögen des Dosierbehälters D das Gesamtvolumen des eindosierten Wassers und der zudosierten Lauge nur geringfügig. Der Dosierbehälter D sollte zweckmäßig während des Dosiervorganges immer nahezu vollständig mit der Flüssigkeit gefüllt werden, damit in dem Dosierbehälter D kein Gasraum verbleibt, in welchen NH₃ schon während des Dosiervorganges entweichen kann. Das der zu untersuchenden Wasserprobe zugeführte Alkalimetallhydroxyd sollte ausreichen, um die Wasserprobe auf einen pH-Wert von mindestens 10,5, vorzugsweise mindestens 12, zu alkalisieren. Vorzugsweise wird die Konzentration der Alkalimetallhydroxydlösung so gewählt, daß ein Verhältnis von zu untersuchendem Wasser zu verdünnter Lauge von 1:4 bis 1:6 zur Erzielung des gewünschten pH-Wertes ausreicht. Geeignet sind beispielsweise 0,1-0,5 molare Natronlaugen.

Von dem Dosierbehälter D führt eine mit einem Ventil V4 versehene Verbindungsleitung L4 zu der Meßzelle M. Von der Meßzelle M führt eine mit einem Ventil V11 versehene Wasserableitung L11 zu der Rohrleitung L0 zurück. Die geschlossene Meßzelle ist in ihrem oberen Bereich mit einer mit einem Ventil V9 versehenen Gaszuleitung L9 mit der Saug/Druckpumpe für Luft SP verbunden. Sie besitzt ferner eine mit einem Ventil V10 versehene Gasableitung L10, welche gewünschtenfalls ihrerseits mit der Saugseite der Saug/Druckpumpe über eine zwischen dem Ventil V5 und der Pumpe SP liegende Abzweigung der Gasleitung L5 verbunden sein kann.

Nach Schließung der Zuleitungsventile V1, V2, V3 und Öffnung des Ventils V4 der Verbindungsleitung L4 wird die Flüssigkeit aus dem Dosierbehälter D durch mittels der Saug/Druckpumpe SP eingedrückte Luft vollständig verdrängt und in die Meßzelle M geleitet. Hierbei sind die Ventile V8, V5 und V10 der Gasleitungen L8, L5 und L10 geöffnet und die Ventile V7, V6 und V9 der Gasleitungen L7, L6 und L9 geschlossen.

Die Meßzelle enthält einen NH₃-sensitiven Gassensor. Es können an sich bekannte handelsübliche Sensoren verwendet werden, welche entweder zur Messung in der Gasphase oder zur Messung in der Flüssigkeitsphase geeignet sind. Je nach Art des verwendeten Sensors ist dieser in der Meßzelle so angebracht, daß er in den die Flüssigkeit aufnehmenden Bereich hineinragt, oder er ist im oberen Bereich der Meßzelle so angebracht, daß er mit der Flüssigkeit nicht in Berührung kommt. Als Meßbereich der Meßzelle wird im Rahmen der vorliegenden Erfindung die räumliche Zone der Meßzelle verstanden, in welcher die Messung stattfindet. Die Größe der Meßzelle kann je nach Art der Anbringung des Sensors variieren. Bei Verwendung eines in der Flüssigphase messenden Sensors braucht das Fassungsvermögen der Meßzelle nur wenig größer als der Dosierbehälter sein. Bei Verwendung eines in der Gasphase messenden Sensors muß die Meßzelle ausreichend groß sein, so daß die aus dem Dosierbehälter kommende Flüssigkeit nur ihren untern Bereich ausfüllt. Beispielsweise ist das Fassungsvermögen der Meßzelle etwa das 1 1/2- bis 2 1/2-fache des Dosierbehälters.

Als Gassensor wird vorzugsweise eine NH₃-sensitive Meßelektrode verwendet. Es können an sich bekannte NH₃-sensitive Meßelektroden eingesetzt werden. Als günstig erweist sich beispielsweise eine Meßelektrode deren elektrischer Widerstand sich in Abhängigkeit von der NH₃-Konzentration ändert. Der Widerstand ist um so größer, je geringer die Ammoniakkonzentration ist. Die widerstands-proportionale Spannung der Meßelektrode kann innerhalb eines mit einem entsprechenden Programm ausgestatteten Mikroprozessors in einen Meßwert umgerechnet werden. Durch Vergleich der in entsprechender Weise mit Eichwässern einer bestimmten Ammonium/Ammoniakkonzentration erhaltenen Meßwerten können die so gewonnenen Meßwerte bei entsprechender Programmgestaltung sofort automatisch in den Ammonium/Ammoniakgehalt des untersuchten Wassers umgerechnet werden.

Die meßbaren Ammoniakkonzentrationen sind abhängig von der Sensibilität der eingesetzten Meßelektrode. Im allgemeinen besitzen die NH₃-sensitiven Meßelektroden eine ausreichende Genauigkeit um Ammoniak/Amonniumkonzentrationen zwischen 10 und 300 ppm in dem zu untersuchenden Wasser quantitativ bestimmen zu können. Qualitative Nachweise sind auch für sehr viel geringere Konzentration, z.B. Konzentrationen ab 0,01 ppm möglich.

Die Meßzelle ist beheizbar und mit einer Temperaturmeß- und -regelvorrichtung T versehen, da das Einhalten von jeweils gleichen Temperaturen für die Reproduzierbarkeit der Meßergebnisse zweckmäßig ist. Insbesondere ist dies wichtig, wenn die NH₃-Messung in dem über der Flüssigkeit befindlichen Gasraum stattfindet, da das Gleichgewicht zwischen in Wasser gelöstem Ammoniak und dem in dem darüber befindlichen Gasraum vorhandnen Ammoniak stark temperaturabhägig ist. Für die Ammoniakbestimmungen in wäßrigen Systemen sind konstante Temperaturen im Bereich zwischen 10 und 50°C gut geeignet. Vorteilhaft ist die Meßzelle zur Beschleunigung der Einstellung einer gleichmäßigen Ammoniakkonzentration im Meßbereich bzw. der Einstellung des Gleichgewichtes zwischen Ammoniakkonzentration in der Flüssigkeit und dem darüber befindlichen Gasraum mit einem Rührer R, vorzugsweise einem Magnetrührer, ausgestattet. Die Flüssigkeit wird in der Meßzelle bei der konstanten Meßtemperatur solange gerührt, bis sich eine konstante Ammoniakkonzentration in dem Meßbereich eingestellt hat und der NH₃-Sensor einen konstant bleibenden Meßwert anzeigt. Je nach Meßtemperatur, Rührgeschwindigkeit und pH-Wert der Lösung kann die Zeitdauer bis zur Einstellung der konstanten Ammoniakkonzentration variieren. Im allgemeinen genügen Verweilzeiten der Lösung in der Meßzelle zwischen 2 und 10 vorzugsweise 3 und 6 Minuten. Sobald der Meßwert über einen vorbestimmten Zeitraum, beispielsweise 20 bis 60 Sekunden konstant geblieben ist, wird die Meßflüssigkeit bei geschlossenem Verbindungsventil V4 und geöffnetem Ableitungsventil V11 mit Druckluft bei geöffnetem Ventil V9 und geschlossenem Ventil V10 aus der Meßzelle ausgeblasen und über die Ableitung L11 in die Rohrleitung L0 zurückgeleitet. Die Meßzelle M wird anschließend gründlich mit Luft zur Beseitigung von NH₃-Restkonzentrationen ausgeblasen. Auch der Dosierbehälter D und die Verbindungsleitung L4 können gegebenenfalls bei geschlossenen Ventilen V1-V3 der Flüssigkeitsleitungen L1-L3 durch Ausblasen mit Luft gereinigt werden. Das Gerät ist sodann zur Messung einer weiteren Wasserprobe funktionsbereit.

Die einzelnen Aggregate des Gerätes sind über einen Mikroprozessor so steuerbar, daß das Ansaugen einer Wasserprobe, der Meßvorgang und das Austreiben der gemessenen Probe automatisch entsprechend einem vorgegebenen Programm ablaufen. Das Gerät kann mit einem einfachen Basic-Programm arbeiten und über ein Terminal in bequemer Weise bedient werden.

Dosierbehälter und Meßzelle sowie die flüssigkeitsführenden Leitungen des Gerätes sind zweckmäßig aus einem korrosions- und alkalibeständigem Material gearbeitet. Geeignet sind beispielsweise Kunststoffe wie PVC und/oder Acrylharze.

Durch die erfindungsgmäße Verwendung von Dosierbehälter und Meßzelle, welche so angeordnet werden können, daß zwischen ihnen als flüssigkeitsführende Verbindung nur ein kurzes das Ventil V₄ enthaltendes starres Leitungsstück L₄ benötigt wird, und die erfindungdgemäße Verwendung von Druckluft zum Transport der Flüssigkeiten durch das Gerät, können Funktionsstörungen, z.B. durch Schlauchbruch, vermieden werden.

Nach einer Ausführungsform des Gerätes, in welcher im Dosierbehälter und in der Meßzelle zweckmäßig Gasein- und -auslässe jeweils zu einer Öffnung zusammengefaßt sind, kann das Gerät modular aus mehreren, z.B. 2-3, unabhängig voneinander austauschbaren Modulen zusammengesetzt sein. Hierbei kann ein Modul Dosierbehälter, Meßzelle und die dazugehörigen Flüssigkeitsleitungen enthalten, ein zweites Modul die Saug/Druckpumpe und die Gasleitungen sowie den Mikroprozessor enthalten und ein allfälliges drittes Modul ein handelsübliches Terminal darstellen, über welches z.B. Programmänderungen, Funktionsanzeigen usw. möglich sind.

Sobald durch Messen entsprechender Eichlösungen das Gerät kalibriert ist, kann damit in einfacher Weise eine halbkontinuierliche automatische Überwachung des Ammonium/Ammoniakgehaltes eines Wassers durchgeführt werden. Der Ablauf der einzelnen Analysen benötigt dabei nur wenige Minuten, beispielsweise zwischen 2 und 10, vorzugsweise 3 und 6 Minuten. Die Meßergebnisse werden durch andere im Wasser vorhandene Ionen nicht gestört.

Das nachfolgende Beispiel soll die Erfindung näher erläutern ohne jedoch ihren Umfang zu beschränken.

### Beispiel 1:

### Ammoniumbestimmung in Wasser

Die Ammoniumbestimmung wurde in der vorstehend beschriebenen Meßapparatur, welche als Gassensor eine ammoniumsensitive Meßelektrode (Gassensor TGS 824 der Fa. Figaro Engineering Inc.) enthielt, wie folgt ausgeführt:

### a) Erstellung einer Eichkurve.

Es wurden 5 Lösungen mit bekannten definierten NH₄⁺-Gehalten im Konzentrationsbereich von 9,7 bis 278 mg/l hergestellt. Die Überprüfung des Ammoniumgehaltes erfolgte mittels Ionenchromatographie.

Von jeder dieser Lösungen wurden 10 Proben in dem Meßgerät vermessen. Dazu wurden jeweils 17 ml der Lösung und 3 ml 0,5-molare wäßrige Natriumhydroxidlösung in den Dosierbehälter eingeführt und vermischt. Der pH-Wert der alkalisierten Proben war immer über pH 12. Aus dem Dosierbehälter wurde die Meßprobe in die Meßzelle überführt und dort bei konstanter Temperatur von 35 °C gerührt, und die Ammoniakkonzentration gemessen. Sobald der Meßwert über einen Zeitraum von 30 sek. konstant blieb, wurde dieser Meßwert von dem Gerät aufgezeichnet und programmgemäß umgerechnet. Die Lösung wurde sodann mittels Druckluft aus der Meßzelle entfernt. Ein Meßvorgang dauerte im Schnitt 3-5 min. Die erhaltenen Mittelwerte aus den jeweils 10 Einzelmessungen wiesen eine maximale Standardabweichung von ±4 % auf. Die aus den Meßwerten berechneten Ammoniumkonzentrationen stimmten mit den vorher ionenchromatographisch ermittelten Werten im Rahmen dieser Standardabweichung überein.

Aus den Meßwerten wurde unter Zuhilfenahme der Sensorcharakteristik eine Eichgerade berechnet, welche einen Korrelationskoeffizienten von -0,994 aufwies.

### b) Bestimmung des Ammoniumgehaltes eines Wassers mit unbekannter Ammoniumkonzentration im Bereich zwischen 10-300 mg/l.

Unter den unter a) beschrieben Versuchsbedingungen wurden 7 Proben des zu untersuchenden Wassers in dem Gerät vermessen. Der Mittelwert der Einzelmessungen wies eine Standardabweichung von ± 2 % auf. Aus dem Mittelwert der Meßwerte errechnete sich eine Ammoniumkonzentration von 46,0 mg/l. Bei einer ionenchromatographischen Überprüfung wurde eine Ammoniumkonzentration von 47,5 mg/l gefunden.

Die Messungen wurden mit Wassern, welche 3 g/l an Neutralsalzen (Natriumchlorid und Kalziumchlorid) enthielten, wiederholt. Das Meßergebnis wurde durch die Anwesenheit dieser Neutralsalze nicht beeinflußt.

## Patentansprüche

1. Gerät zur Bestimmung von in Wasser gelösten Gasen und/oder in Gase überführbaren Substanzen, gekennzeichnet durch
a) einen verschließbaren Dosierbehälter (D) zur Aufnahme einer Probe des zu untersuchenden Wassers und allfälliger Reagenzlösungen, welcher mit Füllstandsanzeigern oder Füllstandssensoren versehen ist, in seinem unteren Bereich einen Flüssigkeitseinlaß und einen Flüssigkeitsauslaß besitzt, und in seinem oberen Bereich einen Einlaß und einen Auslaß für Gas, insbesondere Luft oder ein Inertgas, besitzt,
b) mit dem Flüssigkeitseinlaß des Dosierbehälters (D) verbundene mit Ventilen (V1, V2, V3) versehene Flüssigkeitszuleitungen (L1, L2, L3) für die zu untersuchende Wasserprobe, allfällige Reagenzlösungen und Eichlösungen,
c) eine verschließbare, mit einer Temperaturmeß- und -Regeleinrichtung (T) und einem Sensor (S) für das zu bestimmende Gas versehene Meßzelle (M), welche einen zur Aufnahme der Flüssigkeitsmenge aus dem Dosierbehälter eingerichteten Bereich mit einem Flüssigkeitseinlaß und einem Flüssigkeitsauslaß besitzt und mit einem Einlaß und einen Auslaß für Gas, insbesondere Luft oder ein Inertgas versehen ist,
d) eine mit einem Ventil (V4) versehene Flüssigkeitsleitung (L4), welche den Flüssigkeitsauslaß des Dosierbehälters (D) mit dem Flüssigkeitseinlaß der Meßzelle (M) verbindet,
e) eine Saugdruckpumpe (SP) für Luft oder ein Inertgas, welche über jeweils mit einem Ventil (V5, V6) versehene Gaszu- und -ableitungen (L5, L6) be- bzw. entlüftbar ist, und welche auf ihrer Saugseite über eine von der Gaszuleitung (L5) zwischen deren Ventil (V5) und der Saug/Druckpumpe (SP) abzweigende mit einem Ventil (V7) versehene Gasleitung (L7) mit dem Gasauslaß des Dosierbehälters (D) und auf ihrer Druckseite über von der Gasableitung (L6) zwischen deren Ventil (V6) und der Saugdruckpumpe (SP) abzweigende jeweils mit Ventilen (V8, V9) versehenen Gasleitungen (L8, L9) mit dem Gaseinlaß des Dosierbehälters (D) und dem Gaseinlaß der Meßzelle (M) verbunden ist,
f) eine mit dem Gasauslaß der Meßzelle (M) verbundene mit einem Ventil (V10) versehene Gasableitung (L10),
g) eine mit dem Flüssigkeitsauslaß der Meßzelle (M) verbundene mit einem Ventil (V11) versehene Flüssigkeitsableitung (L11).

2. Gerät nach Anspruch 1, dadurch gekennzeichnet, daß der Gassensor (S) mit einem Aufzeichnungs- und/oder Umrechnungsgerät verbunden ist, welches die von dem Gassensor (S) gemessene Konzentration des zu bestimmenden Gases aufzeichnen und/oder im Vergleich mit Eichwerten daraus die Konzentration des betreffenden Gases oder der dieses Gas generierenden Substanz in der untersuchten Wasserprobe berechnen kann.

3. Gerät nach Anspruch 1 oder 2, dadurch gekennzeichnet, daß die Ventile (V1-V11) elektronisch gesteuerte Ventile sind.

4. Gerät nach einem der vorhergehenden Ansprüche, dadurch gekennzeichnet, daß die Meßzelle (M) mit einem Rührer (R) versehen ist.

5. Gerät nach einem der vorhergehenden Ansprüche, dadurch gekennzeichnet, daß der Sensor (S) in den zur Aufnahme der Flüssigkeit eingerichteten Bereich der Meßzelle hineinragt.

6. Gerät nach einem der Ansprüche 1 bis 4, dadurch gekennzeichnet, daß die Meßzelle (M) einen zur Aufnahme der Flüssigkeitsmenge aus dem Dosierbehälter eingerichteten unteren Bereich mit einem Flüssigkeitseinlaß und einem Flüssigkeitsauslaß besitzt und einen zur Aufnahme von Gas, insbesondere Luft oder einem Inertgas, bestimmten oberen Bereich mit einem Gaseinlaß und einem Gasauslaß, in welchem ein Sensor (S) für das zu bestimmende Gas so angebracht ist, daß er nicht mit der zu untersuchenden Flüssigkeit in Berührung kommt, besitzt.

7. Gerät nach einem der vorhergehenden Ansprüche, dadurch gekennzeichnet, daß der Sensor (S), ein NH₃-sensitiver Sensor, vorzugsweise eine NH₃-sensitive Meßelektrode ist.

8. Gerät nach einem der vorhergehenden Ansprüche, dadurch gekennzeichnet, daß die Meßzelle (M) noch mit weiteren in ihren zur Aufnahme der Flüssigkeit eingerichteten Bereich hineinragenden Sensoren für weitere in dem zu untersuchenden Wasser enthaltene Stoffe versehen ist.

9. Gerät nach Anspruch 3 zur automatischen halbkontinuierlichen Durchführung der Bestimmung, dadurch gekennzeichnet, daß die Ventile (V1-V11) und die Saugdruckpumpe (SP) über einen elektronischen Mikroprozessor derart steuerbar sind, daß in sich wiederholenden Zyklen jeweils zunächst das zu untersuchende Wasser und eine allfällige Reagenzlösung bis zu vorbestimmten Füllständen in den Dosierbehälter (D) eingesaugt werden, sodann die Flüssigkeit aus dem Dosierbehälter (D) in die Meßzelle (M) gedrückt wird, darin verbleibt, bis der Gassensor (S) eine konstante Konzentration des zu messenden Gases anzeigt und sodann die Flüssigkeit aus der Meßzelle (M) ausgedrückt und diese mit Luft oder dem Inertgas gespült wird.

10. Gerät nach Anspruch 9, geeignet zur automatischen halbkontinuierlichen Durchführung der Bestimmung in fließendem Wasser, dadurch gekennzeichnet, daß es eine mit einer Förderpumpe (FP) versehene Rohrleitung (LO) mit einem Wassereinlaß und einem Wasserauslaß zur Durchleitung eines Teiles des fließenden Wassers besitzt, von welcher vor der Förderpumpe (FP) die Zuleitung (L1) zum Dosierbehälter (D) abzweigt, und in welche hinter der Förderpumpe (FP) die mit dem Flüssigkeitsauslaß der Meßzelle (M) verbundene Flüssigkeitsableitung (L11) einmündet.

11. Verfahren zur halbkontinuierlich und automatisch mit dem Gerät gemäß Anspruch 1 durchführbaren Bestimmung des Ammoniak- und/oder Ammoniumgehaltes in Wasser, dadurch gekennzeichnet, daß man
a') das zu untersuchende Wasser über eine mit einem Ventil versehene Zuleitung in einen verschließbaren mit Flüssigkeits- und Gasein- und -auslässen versehenen Dosierbehälter bis zu einem einer gewünschten Probenmenge entsprechenden Füllstand einführt,
b') in den das Wasser enthaltenden Dosierbehälter über eine weitere mit einem Ventil versehende Zuleitung eine verdünnte wäßrige Alkalimetallhydroxidlösung einführt bis zur Erreichung eines Gesamtfüllstandes, welcher einer zur Einstellung eines pH-Wertes von mindestens 10,5, vorzugsweise mindestens 12, ausreichenden Zugabe an Alkalimetallhydroxid entspricht,
c') die alkalisch gestellte Wasserprobe aus dem Dosierbehälter über eine mit einem Ventil versehene Verbindungsleitung in eine mit Flüssigkeits- und Gasein- und -auslässen versehene Meßzelle überführt, in welcher ein NH₃-sensitiver Sensor, vorzugsweise eine NH₃-sensitive Meßelektrode, angebracht ist,
d') in der Meßzelle eine konstante Temperatur im Bereich von 10 bis 50 °C aufrechterhält und in dem Meßbereich der Meßzelle eine konstante NH₃-Konzentration sich einstellen läßt,
e') die konstante NH₃-Konzentration in dem Meßbereich mißt und anschließend die alkalisierte Wasserprobe aus der Meßzelle abführt und
f') aus dem gemessenen Wert durch Vergleich mit unter gleichen Bedingungen erhaltenen Eichwerten den Ammoniak und/oder Ammoniumgehalt des Wassers bestimmt.

12. Verfahren nach Anspruch 11, dadurch gekennzeichnet, daß man die alkalisch gestellte Wasserprobe aus dem Dosierbehälter über eine mit einem Ventil versehene Verbindungsleitung in den unteren Bereich einer mit Flüssigkeits- und Gasein- und -auslässen versehenen Meßzelle überführt, in deren oberem Bereich ein NH₃-sensitiver Sensor, vorzugsweise eine NH₃-sensitive Meßelektrode, so angebracht ist, daß sie nicht mit der Flüssigkeit in Berührung kommt, in der Meßzelle eine konstante Temperatur im Bereich von 10 bis 50 °C aufrechterhält und in dem Gasraum der Meßzelle eine konstante NH₃-Konzentration sich einstellen läßt, und die konstante NH₃-Konzentration in dem Gasraum der Meßzelle mißt und anschließend die alkalisierte Wasserprobe aus der Meßzelle abführt.

13. Verfahren nach Anspruch 11 oder 12, dadurch gekennzeichnet, daß das zu untersuchende Wasser und die Alkalimetallhydroxidlösung mit Hilfe einer Saug/Druckpumpe für Luft in den Dosierbehälter bis zum vorgegebenen Füllstand angesaugt werden, die Flüssigkeit dann mit Hilfe der Saug/ Druckpumpe aus dem Dosierbehälter in die Meßzelle gedrückt wird, und nach Erreichen einer konstanten Ammoniakkonzentration in dem Meßbereich der Meßzelle die Flüssigkeit aus der Meßzelle gedrückt und diese mit Luft gespült wird, wobei die Saug/Druckpumpe über mit Ventilen versehenen Gasleitungen mit dem Gaseinlaß und dem Gasauslaß des Dosierbehälters und dem Gaseinlaß der Meßzelle sowie mit der Außenluft verbunden ist, und beim Ansaugen der Flüssigkeit in den Dosierbehälter die Ventile der jeweiligen Flüssigkeitszuleitungen zum Dosierbehälter und das Ventil der Gasableitung des Dosierbehälters geöffnet und das Ventil der Gaszuleitung zum Dosierbehälter und das Ventil der Verbindungsleitung zur Meßzelle geschlossen sind, und beim Drücken der Flüssigkeit aus dem Dosierbehälter in die Meßzelle, die Ventile der Flüssigkeitszuleitungen zum Dosierbehälter, der Gasableitung des Dosierbehälters und der Gaszuleitung zur Meßzelle und der Flüssigkeitsauslaß der Meßzelle geschlossen und die Ventile der Verbindungsleitung zur Meßzelle und der Gaszuleitung zum Dosierbehälter und der Gasauslaß der Meßzelle geöffnet sind, und beim Ausdrücken der Flüssigkeit aus der Meßzelle das Ventil der Verbindungsleitung zum Dosierbehälter geschlossen und der Flüssigkeitsauslaß der Meßzelle und das Ventil der Gaszuleitung zur Meßzelle geöffnet sind.

14. Verfahren nach Anspruch 13, dadurch gekennzeichnet, daß der Transport des zu untersuchenden Wassers automatisch durch eine elektronische Steuerung der Ventile und der Druck/Saugpumpe erfolgt.

## Claims

1. Equipment for the determination of gases and/or substances transferrable into gases, dissolved in water characterised by
a) a lockable dosing vessel (D), to tee a sample of the water to be examined and any reagent solutions, fitted with level indicators or level sensors, having in its bottom section a liquid inlet and a liquid outlet and in its top section one inlet and one outlet for gas, in particular air or an inert gas,
b) liquid Supply pipes (L1, L2, L3) fitted with valves (V1, V2, V3) connected to the liquid inlet of the dosing vessel (D) for the water sample to be examined, any reagent solutions and calibration solutions,
c) a lockable measuring cell (M) fitted with a temperature measurement and adjustment installation (T) and a sensor (S) for the gas to be analyzed which has a section intended to take the quantity of liquid from the dosing vessel with a liquid inlet and a liquid outlet and which has an inlet and an outlet for gas, in particular air or an inert gas,
d) a liquid pipe (L4) fitted with a valve (V4) which connects the liquid outlet of the dosing vessel (D) to the liquid inlet of the measuring cell (M),
e) a suction pressure pump (SP) for air or an inert gas, which can be ventilated or vented via gas supply and drainage pipes (L5, L6) each fitted with a valve (V5, V6) and which is connected to the gas outlet of the dosing vessel (D) on the suction side via a gas pipe (L7) fitted with a valve (V7), branching from the gas supply pipe (L5) between its valve and the suction/pressure pump (SP), and connected to the gas inlet of the dosing vessel (D) and the gas inlet of the measuring cell (M) on its pressure side via gas pipes (L8, L9) each fitted with valves (V8, V9), branching from the gas drainage pipe (L6) between its valve (V6) and the suction pressure pump (SP),
f) a gas drainage pipe (L10) connected to the gas outlet of the measuring cell (M) fitted with a valve (V10),
g) a liquid drainage pipe (L11) connected to the liquid outlet of the measuring cell (M) and fitted with a valve (V11).

2. Equipment per claim 1, characterised by the fact that the gas sensor (S) is connected to a recording and/or conversion device which can record the concentration of the gas to be determined measured by the gas sensor (S) and/or can calculate the concentration of the gas concerned or the substance generating this gas in the water sample examined in comparison with calibration values.

3. Equipment per claim 1 or 2, characterised by the fact that the valves (V1-V11) are electronically controlled valves.

4. Equipment per one of the previous claims, characterised by the fact that the measuring cell (M) is fitted with a mixer (R).

5. Equipment per one of the previous claims, characterised by the fact that the sensor (S) projects into the area of the measuring cell intended to take the liquid.

6. Equipment per one of the claims 1-4, characterised by the fact that the measuring cell (M) has a bottom section intended to take the quantity of liquid from the dosing vessel, with one liquid inlet and one liquid outlet, and a top section intended to take gas, in particular air or an inert gas, with one gas inlet and one gas outlet, in which a sensor (S) for the gas to be determined is arranged such that it does not come into contact with the liquid to be examined.

7. Equipment per one of the previous claims, characterised by the fact that the sensor (S) is an NH₃ sensitive sensor, preferably an NH₃ sensitive measuring electrode.

8. Equipment per one of the previous claims, characterised by the fact that the measuring cell (M) is fitted with further sensors projecting into the section intended to take the liquid, these sensors being for further substances contained in the water to be examined.

9. Equipment per claim 3, for automatic semi-continuous analysis operation, characterised by the fact that the valves (V1-V11) and the suction pressure pump (SP) can be controlled via an electronic microprocessor, such that the water to be examined and any reagent solution required can be drawn into the dosing vessel (D) up to preset levels, in recurrent cycles, so that the liquid from the dosing vessel (D) is forced into the measuring cell (M) and stays there until the gas sensor (S) displays a constant concentration of the gas to be measured and then the liquid is forced out of the measuring cell (M) which is then flushed with air or the inert gas.

10. Equipment per claim 9, suitable for automatic semi-continuous analysis operation in running water, characterised by the fact that it has a pipe (LO) fitted with a feed-pump (FP) with one water inlet and one water outlet to take part of the running water, from which the supply pipe (L1) to the dosing vessel (D) branches off before the feed pump (FP) and into which, after the feed pump (FP), the liquid drainage pipe (L11) connected to the measuring cell (M) liquid outlet opens.

11. Process for the semi-continuous and automatic analysis, using equipment per claim 1, of the ammonia and/or ammonium content in water, characterised by the fact that
a') the water to be examined is introduced, via a supply pipe fitted with a valve, into a lockable dosing vessel fitted with liquid and gas inlets and outlets, up to a level corresponding to a desired sample quantity,
b') a dilute aqueous alkali metal hydroxide solution is introduced into the dosing vessel containing the water via a further supply pipe fitted with a valve, until a total level is reached which corresponds to the addition of alkali metal hydroxide sufficient to produce a pH of at least 10.5 and preferably of at least 12,
c') the alkalised water sample is transferred from the dozing vessel via a connection pipe fitted with a valve into a measuring cell fitted with liquid and gas inlets and outlets in which an NH₃ sensitive sensor, preferably an NH₃ sensitive measuring electrode, is located,
d') a constant temperature in the range 10-50°C is maintained in the measuring cell and a constant NH₃ concentration is allowed to form in the measuring section of the measuring cell,
e') the constant NH₃ concentration is measured in the measuring section and then the alkalised water sample is drained from the measuring cell, and
f') the ammonia and/or ammonium content of the water is determined from the measured value by comparison with calibration values obtained under the same conditions.

12. Process per claim 11, characterised by the fact that the alkalised water sample is transferred from the dosing vessel via a connection pipe fitted with a value into the bottom section of a measuring cell fitted with liquid end gas inlets and outlets, in the top section of which, an NH₃ sensitive sensor, preferably an NH₃ sensitive measuring electrode, is located such that it does not come into contact with the liquid, a constant temperature of 10-50 °C is maintained in the measuring cell and a constant NH₃ concentration is allowed to form in the gas chamber of the measuring cell, the constant NH₃ concentration in the gas chamber of the measuring cell is measured and then the alkalised water sample from the measuring cell is drained off.

13. Process per claim 11 or 12, characterised by the fact that the water to be examined and the alkali metal hydroxide solution is drawn up to the set level in the dosing vessel with the help of a suction/pressure pump for air, the liquid is then forced from the dozing vessel into the measuring cell with the help of the suction/pressure pump, and once a constant ammonia concentration has been reached in the measuring section of the measuring cell, the liquid is forced out of the measuring cell which is flushed with air, whereby the suction/pressure pump is connected to the gas inlet and the gas outlet of the dozing vessel and the gas inlet of the measuring cell and also the external air via gas pipes fitted with valves, and when the liquid is drawn into the dosing vessel, the valves of the various liquid supply pipes to the dosing vessel and the valve of the gas drainage pipe of the dozing vessel are opened and the valve of the gas supply pipe to the dozing vessel and the valve of the connection pipe to the measuring cell are closed, and when the liquid is forced out of the dozing vessel into the measuring cell, the valves of the liquid supply pipes to the dozing vessel, the gas drainage pipe of the dosing vessel and the gas supply pipe to the measuring cell and the liquid outlet of the measuring cell are closed, and the valves of the connection pipe to the measuring cell and the gas supply pipe to the dozing vessel and the gas outlet of the measuring cell are opened, and when the liquid is forced out of the measuring cell, the valve of the connection pipe to the dozing vessel is closed and the liquid outlet of the measuring cell and the valve of the gas supply pipe to the measuring cell are opened.

14. Process per claim 13, characterized by the fact that the water to be examined is transported automatically by electronic control of the valves and the pressure/suction pump.

## Revendications

1. Appareil de dosage de gaz et/ou de substances convertibles en gaz dissous dans l'eau, caractérisé par:
a) Un réceptacle de dosage obturable (D) pour recevoir un échantillon de l'eau à analyser et de solutions réactives éventuelles, lequel réceptacle comporte des indicateurs de niveau ou des capteurs de niveau, possède dans sa partie inférieure une entrée et une sortie de liquide et dans sa partie supérieure une entrée et une sortie de gaz, en particulier de l'air ou un gaz inerte;
b) Des conduites d'amenée de liquide (L1, L2, L3) reliées à l'entrée de liquide du réceptacle de dosage (D) et dotées de soupapes (V1, V2, V3) pour l'échantillon d'eau à analyser, les solutions réactives éventuelles et les solutions d'étalonnage;
c) Une cellule de mesure (M) obturable, dotée d'un dispositif de mesure et de réglage de la température (T) et d'un capteur (S) pour le gaz à doser, laquelle cellule comporte une zone agencée pour recevoir la quantité de liquide du réceptacle de dosage avec une entrée et une sortie de liquide et une entrée et une sortie de gaz, en particulier de l'air ou un gaz inerte;
d) Une conduite de liquide (L4) dotée d'une soupape (V4) qui relie la sortie de liquide du réceptacle de dosage (D) à l'entrée de liquide de la cellule de mesure (M);
e) Une pompe d'aspiration/refoulement (SP) pour l'air ou un gaz inerte, qui peut être alimentée en air ou évacuée respectivement via des conduites d'amenée et d'évacuation de gaz (L5, L6) dotées d'une soupape (V5, V6), et qui, sur son côté d'aspiration, est reliée, via une conduite de gaz (L7) dotée d'une soupape (V7) dérivée de la conduite d'amenée de gaz (L5) entre sa soupape (V5) et la pompe d'aspiration/compression (SP), àla sortie de gaz du réceptacle de dosage (D) et, sur son côté de refoulement, est reliée, via des conduites de gaz (L8, L9) dotées respectivement de soupapes (V8, V9) et dérivées de la conduite d'évacuation de gaz (L6) entre sa soupape (V6) et la pompe d'aspiration et de refoulement (SP), àl'entrée de gaz du réceptacle de dosage (D) et àl'entrée de gaz de la cellule de mesure (M),
f) une conduite d'évacuation de gaz dotée (L10) d'une soupape (V10) reliée à la sortie de gaz de la cellule de mesure (M),
g) une conduite d'évacuation de liquide (L11) dotée d'une soupape (V11) et reliée à la sortie de liquide de la cellule de mesure (M).

2. Appareil selon la revendication 1, caractérisé en ce que le capteur de gaz (S) est relié à un appareil d'enregistrement et/ou de conversion qui peut enregistrer la concentration du gaz à doser mesurée par le capteur de gaz (S) et/ou peut en déduire, par comparaison avec des valeurs d'étalonnage, la concentration du gaz concerné ou de la substance générant ce gaz dans l'échantillon d'eau à analyser.

3. Appareil selon la revendication 1 ou 2, caractérisé en ce que les soupapes (V1-V11) sont des soupapes à commande électronique.

4. Appareil selon l'une quelconque des revendications précédentes, caractérisé en ce que la cellule de mesure (M) est dotée d'un agitateur (R).

5. Appareil selon l'une quelconque des revendications précédentes, caractérisé en ce que le capteur (S) fait saillie dans la zone de la cellule de mesure agencée pour recevoir le liquide.

6. Appareil selon l'une quelconque des revendications 1 à 4, caractérisé en ce que la cellule de mesure (M) comporte une zone inférieure agencée pour recevoir la quantité de liquide du réceptacle de dosage avec une entrée et une sortie de liquide et comporte également une zone supérieure déterminée pour recevoir le gaz, en particulier de l'air ou un gaz inerte, avec une entrée et une sortie de gaz, zone dans laquelle un capteur (S) est agencé pour le gaz à doser en sorte qu'il ne vienne pas en contact avec le liquide àanalyser.

7. Appareil selon l'une quelconque des revendications précédentes, caractérisé en ce que le capteur (S) est un capteur sensible au NH₃, de préférence une électrode de mesure sensible au NH₃.

8. Appareil selon l'une quelconque des revendications précédentes, caractérisé en ce que la cellule de mesure (M) est encore dotée d'autres capteurs faisant saillie dans sa zone agencée pour la réception du liquide pour d'autres substances contenues dans l'eau à analyser.

9. Appareil selon la revendication 3 permettant de réaliser le dosage de manière automatique en semi-continu, caractérisé en ce que les soupapes (V1, V11) et la pompe d'aspiration/refoulement (SP) peuvent être commandées via un microprocesseur électronique de telle sorte qu'on aspire, au cours de cycles répétitifs, respectivement, tout d'abord l'eau à analyser et une solution réactive éventuelle dans le réceptacle de dosage (D) jusqu'à des niveaux de remplissage prédéterminés, qu'on refoule ensuite le liquide du réceptacle de dosage (D) dans la cellule de mesure (M), qu'on l'y maintienne jusqu'à ce que le capteur de gaz (S) indique une concentration constante du gaz à doser et qu'on refoule ensuite le liquide hors de la cellule de mesure (M) et qu'on rince celle-ci par de l'air ou le gaz inerte.

10. Appareil selon la revendication 9 convenant àla réalisation automatique en semi-continu du dosage dans de l'eau courante, caractérisé en ce qu'il possède une conduite tubulaire (LO) dotée d'une pompe d'alimentation (FP) avec une entrée et une sortie d'eau pour acheminer une partie de l'eau courante, conduite àpartir de laquelle la conduite d'amenée (L1) menant au réceptacle de dosage (D) diverge devant la pompe d'alimentation (FP) et dans laquelle la conduite d'évacuation de liquide (L11) reliée à la sortie de liquide de la cellule de mesure (M) débouche derrière la pompe d'alimentation (FP).

11. Procédé de dosage de la teneur en ammoniac et/ou en ammonium dans de l'eau réalisable en semi-continu et en automatique avec l'appareil selon la revendication 1, caractérisé en ce que
a') on introduit l'eau à analyser via une conduite d'amenée dotée d'une soupape dans un réceptacle de dosage obturable doté d'entrées et de sorties de liquide et de gaz jusqu'à un niveau de remplissage correspondant à une quantité d'échantillonnage souhaitée,
b') on introduit dans le réceptacle de dosage contenant l'eau, via une autre conduite d'amenée dotée d'une soupape, une solution d'hydroxyde de métal alcalin aqueuse diluée jusqu'à atteindre un niveau de remplissage total, qui correspond à une addition d'hydroxyde de métal alcalin suffisante pour ajuster une valeur du pH d'au moins 10,5, de préférence d'au moins 12,
c') on transfère l'échantillon d'eau alcalinisé du réceptacle de dosage à une cellule de mesure dotée d'entrées et de sorties de liquide et de gaz via une conduite de communication dotée d'une soupape, cellule dans laquelle on a agencé un capteur sensible au NH₃, de préférence une électrode de mesure sensible au NH₃,
d') on maintient dans la cellule de mesure une température constante dans la plage de 10 à 50°C et on laisse dans la zone de mesure de la cellule de mesure s'instaurer une concentration de NH₃ constante,
e') on mesure la concentration de NH₃ constante de la zone de mesure et ensuite on évacue l'échantillon d'eau alcalinisé de la cellule de mesure, et
f') on détermine à partir de la valeur mesurée, par comparaison avec des valeurs d'étalonnage obtenues dans les mêmes conditions, la teneur en ammoniac et/ou en ammonium de l'eau.

12. Procédé selon la revendication 11, caractérisé en ce que l'on transfère l'échantillon d'eau alcalinisé, via une conduite de communication dotée d'une soupape, du réceptacle de dosage (D) dans la zone inférieure d'une cellule de mesure dotée d'entrées et de sorties de liquide et de gaz, dans la zone supérieure de laquelle on a agencé un capteur sensible au NH₃, de préférence une électrode de mesure sensible au NH₃, en sorte qu'il ne vienne pas en contact avec le liquide, on maintient dans la cellule de mesure une température constante dans la plage de 10 à 50°C et on laisse s'instaurer dans la chambre à gaz de la cellule de mesure une concentration de NH₃ constante, on mesure la concentration de NH₃ constante de la chambre à gaz de la cellule de mesure et ensuite on évacue l'échantillon d'eau alcalinisée de la cellule de mesure.

13. Procédé selon la revendication 11 ou 12, caractérisé en ce que l'eau à analyser et la solution d'hydroxyde de métal alcalin sont aspirées à l'aide d'une pompe d'aspiration/refoulement d'air dans le réceptacle de dosage jusqu'à atteindre le niveau prédéterminé, le liquide est ensuite refoulé à l'aide de la pompe d'aspiration/refoulement du réceptacle de dosage dans la cellule de mesure, et, une fois qu'on a atteint une concentration en ammoniac constante dans la zone de mesure de la cellule de mesure, le liquide est refoulé de la cellule de mesure qui est balayée à l'aide d'air, la pompe d'aspiration/refoulement étant reliée via des conduites de gaz dotées de soupapes à l'entrée de gaz et à la sortie de gaz du réceptacle de dosage et à l'entrée de gaz de la cellule de mesure ainsi qu'à l'air extérieur, et, lors de l'aspiration du liquide dans le réceptacle de dosage, les soupapes des conduites d'amenée de liquide respectives au réceptacle de dosage et la soupape de la conduite d'évacuation de gaz du réceptacle de dosage sont ouvertes et la soupape de la conduite d'amenée de gaz au réceptacle de dosage et la soupape de la conduite de communication avec la cellule de mesure sont fermées, tandis que, lors du refoulement du liquide du réceptacle de dosage dans la cellule de mesure, les soupapes des conduites d'amenée de liquide au réceptacle de dosage, à la conduite d'évacuation de gaz du réceptacle de dosage et à la conduite d'amenée de gaz à la cellule de mesure et à la sortie de liquide de la cellule de mesure sont fermées et les soupapes de la conduite de communication avec la cellule de mesure et de la conduite d'amenée de gaz au réceptacle de dosage et la sortie de gaz de la cellule de mesure sont ouvertes, et lors du refoulement du liquide de la cellule de mesure, la soupape de la conduite de communication avec le réceptacle de dosage est fermée et la sortie de liquide de la cellule de mesure et la soupape de la conduite d'amenée de gaz vers la cellule de mesure sont ouvertes.

14. Procédé selon la revendication 13, caractérisé en ce que le transport de l'eau à analyser se fait automatiquement par commande électronique des soupapes et de la pompe d'aspiration et de refoulement.
